# EUROPÄISCHE PATENTSCHRIFT

(11) **EP 1 079 839 B1**
(45) Veröffentlichungstag und Bekanntmachung des Hinweises auf die Patenterteilung: **08.09.2004**
(21) Anmeldenummer: 99923478.4
(22) Anmeldetag: 28.04.1999
(51) Int. Cl.: A61K 31/70, A61P 11/00

(54) **ISOMALT ALS WIRKSTOFF ENTHALTENDES ERKÄLTUNGSMITTEL**
THERAPEUTIC ANTI-COLD AGENT CONTAINING ISOMALT AS AN ACTIVE INGREDIENT
AGENT THERAPEUTIQUE CONTRE LE RHUME, CONTENANT DE L'ISOMALT COMME PRINCIPE ACTIF

(30) Priorität: 28.04.1998 DE 19818842; 20.10.1998 US 175831
(43) Veröffentlichungstag der Anmeldung: 07.03.2001
(73) Patentinhaber: SÜDZUCKER AKTIENGESELLSCHAFT MANNHEIM/OCHSENFURT, 68165 Mannheim (DE)
(72) Erfinder: KUNZ, Markwart, D-67550 Worms (DE); DÖRR, Tillman, D-67591 Hohen-Sülzen (DE); FRITZSCHING, Bodo, D-69469 Weinheim (DE); KLINGEBERG, Michael, D-67549 Worms (DE); KOZIANOWSKI, Gunhild, D-67261 Grünstadt (DE); RAPP, Knut, M., D-67591 Offstein (DE); KOWALCZYK, Jörg, D-67248 Bockenheim (DE); STRÄTER, Peter, J., D-69123 Heidelberg (DE)
(74) Vertreter: Schrell, Andreas, Dr.
(86) Internationale Anmeldenummer: PCT/EP1999/002866
(87) Internationale Veröffentlichungsnummer: WO 1999/055342

(56) Entgegenhaltungen:
- EP-A- 0 354 442
- EP-A- 0 710 484
- WO-A-91/18091
- WO-A-97/30598
- DATABASE WPI Section Ch, Week 9718 Derwent Publications Ltd., London, GB; Class B03, AN 97-197208 XP002113901 & JP 09 052834 A (TAKEDA SHOKUHIN KOGYO KK), 25. Februar 1997 (1997-02-25)

## Beschreibung

Die vorliegende Erfindung betrifft die Verwendung eines 6-O-α-D-Glucopyranosyl-D-sorbit-(1,6-GPS) und 1-O-α-D-Glucopyranosyl-D-mannit-(1,1-GPM) haltigen Zuckeralkoholgemisches in Arznei-, Lebens- und Genußmitteln als therapeutischer, insbesondere immunstimulierender und/oder antibakterieller Wirkstoff, insbesondere in Verbindung mit Zink, sowie die diese Substanzen enthaltenden Produkte.

Eine weitverbreitete und unangenehme Beschwerden verursachende Krankheit ist die Erkältung. Erkältungen verursachende Mikroorganismen, zum Beispiel Bakterien wie Staphylococcus aureus oder Viren wie Rhinoviren, halten sich in den befallenen Organismen hauptsächlich im Hals-, Rachen- und Nasenraum auf, wo sie im allgemeinen direkt mit Wirkstoffen bekämpft werden können. Geeignete Darreichungsformen für pharmakologische Wirkstoffe sind zum Beispiel Lutschpastillen, Kaugummis oder Komprimate. Derartige Darreichungsformen sind dadurch gekennzeichnet, daß es sich um feste Formulierungen handelt, die sich langsam im Hals- und Rachenraum auflösen und dadurch die Mikroorganismen, die sich oberflächlich an beziehungsweise auf den Schleimhäuten befinden, an ihrer Vermehrung und Verbreitung behindern.

Als pharmazeutisch wirksame Substanz zur Bekämpfung Erkältungen hervorrufender Mikroorganismen ist Zink bekannt. In einer Studie mit Rhinoviren konnte gezeigt werden, daß die antiviralen Effekte direkt von der Menge an ungebundenen Zn²⁺-Ionen abhängen (Merluzzi et al., in: Research Communications in Chemical Pathology and Pharmacology Vol. 66, (1989) 3, 425-440). Freie, das heißt unkomplexierte, Zinkionen gelten heute als pharmakologisches Mittel, das die Symptome von Erkältungskrankheiten deutlich sowohl in ihrer Dauer als auch in ihrer Schwere vermindert (Mossad et al., in: Annals of Internal Medicine, Vol. 125 (1996) 2, 81-87 und Godfrey et al., in: Alternative Therapies, Vol. 2, (1996) 6, 63-72). Eine wesentliche Voraussetzung für den beobachteten therapeutischen Effekt von Zink ist die Notwendigkeit, den die Zinkionen enthaltenden pharmazeutischen Träger, zum Beispiel eine Lutschpastille, ab Beginn des Auftretens der Symptome möglichst ununterbrochen, zum Beispiel alle 1,5 bis 2 Stunden, in regelmäßigen Abständen einzunehmen und im Mund zergehen zu lassen. Der Grund dafür liegt darin, daß die Zinkionen topisch im Mund-, Nasen-/Rachenraum wirken.

Diese Art der kontinuierlichen Wirkstoffapplikation im Mundraum bringt jedoch eine Reihe von erheblichen Nachteilen mit sich, da die in den allermeisten Fällen im pharmazeutischen Träger vorhandenen Mono- und Disaccharide die Kariesbildung fördern. Aus diesem Grund ist es vorteilhaft, zahnfreundliche Zuckeraustauschstoffe als Ersatz für den kariesfördernden Zucker einzusetzen. Als Zuckeraustauschstoff zum Einsatz in zinkhaltigen Präparaten sind Sorbit und Mannit bekannt. Die Verwendung von Sorbit und Mannit bringt jedoch den Nachteil mit sich, daß diese sich im Mund- und Rachenraum schneller lösen, so daß die erforderliche möglichst lange und kontinuierliche Einwirkzeit der Zinkionen nicht immer gewährleistet ist. Zudem ist es zum Beispiel aus den US-Patenten 5,409,905 und 5,002,970 bekannt, daß Sorbit und auch Mannit metallkomplexierende Eigenschaften besitzen und auch Zink komplexieren. Aus Godfrey et al. in: The Journal of International Medical Research 20 (1992), 234-246 und Zarembo et al., in: Journal of Pharmaceutical Sciences, Vol. 81, (1992) 2, 128-130, ist es ebenfalls bekannt, daß Mannit und Sorbit zinkkomplexierend wirken und zudem, daß mannit-, sorbit- und zinksalzhaltige Lutschpastillen demgemäß deutlich verringerte Wirksamkeit im Hinblick auf die zeitliche Verkürzung und Verminderung von Erkältungssymptomen aufweisen (Smith et al., in: Antimicrobial Agents and Chemotherapy 33 (1989) 5, 646-648). Die sich bildenden Mannit- und Sorbit-Zinkkomplexe überführen das Zink in eine pharmazeutisch unwirksame Form.

Das der vorliegenden Erfindung zugrundeliegende technische Problem besteht also darin, ein Produkt bereitzustellen, das eine allgemeine Stimulierung der Immunabwehr und der Linderung und Bekämpfung von Erkältungskrankheiten dient, gleichzeitig aber akariogen und brennwertreduziert ist sowie eine durchgängig effiziente Wirkstoff-Freigabe gewährleistet.

Die vorliegende Erfindung löst dieses Problem durch die Bereitstellung einer Verwendung eines 1,6-GPSund 1,1-GPM-haltigen Zuckeralkoholgemisches in Arznei-, Lebens- und Genußmitteln als therapeutischer Wirkstoff, insbesondere als Immunstimulanz und/oder als antimikrobieller Wirkstoff. Überraschenderweise konnte nämlich gezeigt werden, daß ein Gemisch aus 1,6-GPS und 1,1-GPM, insbesondere ein ungefähr äquimolares Gemisch dieser beiden Zuckeralkohole enthaltend 43-57 Gew.-% 1,1-GPM und 57-43 Gew.-% 1,6-GPS (bezogen auf TS, Trockensubstanz), das auch als Isomalt, hydrierte Isomaltulose oder Palatinit® bezeichnet wird, antimikrobiell wirkt, darüber hinaus aber auch brennwert-reduziert und akariogen ist, eine sehr gute Verarbeitbarkeit aufweist sowie langsamer als Sorbit in Lösung geht, so daß eine verlängerte Wirkstoff-Freigabe ermöglicht wird. Überraschenderweise stellte sich dabei auch heraus, daß ein derartiges Gemisch aus 1,6-GPS und 1,1-GPM immunstimulierende Wirkung aufweist und die körpersowie zelleigene Immunität und Resistenz gegenüber Infektionen erhöht.

In besonders vorteilhafter Ausführungsform der vorliegenden Erfindung weist das 1,6-GPS- und 1,1-GPMhaltige Zuckeralkoholgemisch auch 1,1-GPS (1-O-α-D-Glucopyranosyl-D-sorbit) und gegebenenfalls geringe Mengen an hydrierten oder nicht hydrierten Mono-, Di- und Oligosacchariden, Xylit, Erythrit, Maltit, hydrierte Glucose- und Stärkesirupe, Lactit sowie Polydextrose auf. Erfindungsgemäß einsetzbare Zukkeralkoholgemische sind beispielsweise aus der EP 0 625 578 B1 bekannt, die hinsichtlich derartiger Zuckeralkoholgemische und deren Herstellung in den Offenbarungsgehalt dieser Erfindung mit einbezogen ist. Insbesondere betrifft die Erfindung die vorgenannten Zuckeralkoholgemische, wobei diese beispielsweise 10-50 Gew.-% 1,6-GPS, 0,5-20 Gew.-% 1,1-GPS und 30-70 Gew.-% 1,1-GPM (bezogen auf TS) enthalten können. In einer weiteren bevorzugten Ausführungsform können die erwähnten Zuckeralkoholgemische 5-10 Gew.-% 1,6-GPS, 30-40 Gew.-% 1,1-GPS und 45-60 Gew.-% 1,1-GPM (bezogen auf TS) enthalten.

Im Zusammenhang mit der vorliegenden Erfindung wird unter einem Arzneimittel ein hauptsächlich die Gesundheit eines menschlichen oder tierischen Körpers förderndes oder wiederherstellendes Mittel verstanden, das sowohl prophylaktischer als auch heilender Natur sein kann und einen diese Wirkung hervorrufenden therapeutischen Wirkstoff umfaßt. Ein therapeutischer Wirkstoff im Zusammenhang mit der vorliegenden Erfindung kann also der Heilung von Krankheiten und Störungen der Körperfunktionen sowie der Prophylaxe dienen.

Im Zusammenhang mit der vorliegenden Erfindung wird unter einem Lebensmittel ein hauptsächlich der Ernährung des menschlichen oder tierischen Körper dienendes Produkt wie beispielsweise Joghurt, Marmelade, Konfitüre, einschließlich sogenannter "functional foods" verstanden, während unter einem Genußmittel ein hauptsächlich einem angenehmen Geschmacksempfinden dienendes Produkt wie beispielsweise ein Kaugummi, ein Bonbon, eine Karamelle, eine Schokolade, eine Backware, ein Keks, ein Gummibärchen oder ähnliches verstanden wird. Sowohl die genannten Lebens- als auch die Genußmittel können aufgrund ihres Gehalts an therapeutisch wirkenden Stoffen, also 1,1-GPM und 1,6-GPS, vorzugsweise in Kombination mit Zink, auch als Arzneimittel bezeichnet werden.

Wie erwähnt, wirkt das erfindungsgemäß verwendete Gemisch der Zuckeralkohole, insbesondere zusammen mit Zink, als antimikrobieller Wirkstoff beziehungsweise als Wirkstoffgemisch, das heißt das Gemisch weist die nachstehend definierten Eigenschaften eines antimikrobiellen Wirkstoffs auf. Im Zusammenhang mit der vorliegenden Erfindung wird unter einem antimikrobiellen Wirkstoff ein Wirkstoff verstanden, der auf Bakterien und zwar sowohl grampositive als auch gram-negative Bakterien, Protozoen, Viren, Bakteriophagen, Retroviren, Viroide, Hefen, Algen, Pilze und/oder ähnliche Mikroorganismen wirkt, wobei die Wirkung in einer Wachstumsund/oder Vermehrungshemmung und/oder einem Abtöten der Mikroorganismen besteht, also mikrobistatisch und/oder mikrobizid ist. In besonders bevorzugter Ausführungsform der Erfindung weist ein antimikrobieller Wirkstoff insbesondere bakteriostatische und/oder bakterizide und/oder antivirale Wirkung auf. Diese Wirkung entfaltet sich vorzugsweise, insbesondere bei Einsatz des erfindungsgemäßen Wirkstoffs in Lutschpastillen, topisch im Mund- und Rachenraum.

Das erfindungsgemäße verwendete Gemisch der Zuckeralkohole weist, insbesondere zusammen mit Zink, immunstimulierende Wirkung auf, das heißt verbessert insbesondere die körpereigene Abwehrkraft gegen abnormale und körperfremde Einflüsse, insbesondere Parasiten oder bakterielle oder virale Infektionen durch pathogene Keime, einschließlich Krankheitserregern des respiratorischen und Gastrointestinaltraktes und wirkt auch gegen Tumorentstehung. Die immunstimulierende Wirkung zeichnet sich insbesondere durch die Bereitstellung und Ausschüttung von immunreaktiven Stoffen aus. Diese Wirkung entfaltet sich vorzugsweise aufgrund von heruntergeschlucktem, das heißt im Gastrointestinaltrakt befindlichen erfindungsgemäßen Produkt.

In einer besonders bevorzugten Ausführungsform der vorliegenden Erfindung ist vorgesehen, die erfindungsgemäß eingesetzten 1,6-GPS- und 1,1-GPM-haltigen Zuckeralkoholgemische mit Zink, insbesondere anorganischen oder organischen Zinksalzen wie Zinkgluconat oder Zinkacetat, zu kombinieren. Überraschenderweise hat sich gezeigt, daß obgleich -wie aus der Literatur bekannt- Sorbit oder Mannit Zink komplexieren und daher sorbit- oder mannithaltige Produkte mit einem Zinkgehalt keine oder keine wesentliche erkältungsbekämpfende oder immunstimulierende Funktion haben, die erfindungsgemäß einzusetzenden gelösten 1,6-GPS und 1,1-GPM-haltigen Zuckeralkoholgemische, die zusätzlich Zink enthalten, ein ähnliches Komplexierungsverhalten, das auch bei Produkten auf Basis von Saccharose/Glucosemischungen beobachtet wird, zeigen und somit die Zinkionen größtenteils in der unkomplexierten Form vorliegen und demgemäß ihre antimikrobielle und immunstimulierende Funktion entfalten können. Dieser Befund ist unter anderem deshalb als überraschend anzusehen, als daß die Disaccharidalkohole 1,6-GPS und 1,1-GPM Sorbit- beziehungsweise Mannitderivate sind, die erwartungsgemäß Zink in größerem Maße komplexieren müßten. Die Erfindung betrifft demgemäß auch die Verwendung eines Gemisches aus den vorgenannten Zuckeralkoholen und Zinkionen als antimikrobieller Wirkstoff beziehungsweise als antimikrobielle Wirkstoffkombination, insbesondere als Erkältungsmittel, sowie als Immunstimulanz.

Im Zusammenhang mit der vorliegenden Erfindung werden unter freien oder unkomplexierten Zinkionen solche Zinkionen verstanden, die in wäßriger Lösung nur labile Aquokomplexe bilden, wie sie jedes Ion in wäßrigem Medium in Form einer Solvatationsphäre aufweist. Freie oder unkomplexierte Zinkionen sind demgemäß nicht mit anderen Substanzen in Form eines Komplexes assoziiert.

In bevorzugter Ausführungsform der vorliegenden Erfindung beträgt der Anteil an Zink im Zuckeralkoholgemisch 0,5-10 mg Zink pro g Zuckeralkohol, bevorzugt 1-5 mg Zink pro g Zuckeralkohol. Die vorliegende Erfindung betrifft auch die Verwendung der vorgenannten Zuckeralkoholgemische, insbesondere von 1,1-GPM und 1,6-GPS, vorzugsweise in Kombination mit Zink und/oder 1,1-GPS, zur Herstellung eines Arzneimittels, das immunstimulierend und/oder antimikrobiell wirkt.

Die vorliegende Erfindung betrifft demgemäß auch Produkte, insbesondere Arznei-, Lebens- und Genußmittel, die die vorgenannten Zuckeralkoholgemische, insbesondere 1,1-GPM- und 1,6-GPS-haltige Zuckeralkoholgemische in Verbindung mit Zink, insbesondere organischen oder anorganischen Zinksalzen, enthalten. In besonders bevorzugter Ausführungsform der Erfindung beträgt das Gewichts-Verhältnis der in dem Produkt vorhandenen Zuckeralkohole 1,1-GPM und 1,6-GPS nahezu 1:1, das heißt, das in dem Produkt enthaltene Zuckeralkoholgemisch aus 1,1-GPM und 1,6-GPS ist Isomalt. Erfindungsgemäß kann aber auch vorgesehen sein, daß das Zuckeralkoholgemisch aus 1,1-GPM, 1,6-GPS und 1,1-GPS besteht beziehungsweise eine Mischung dieser Zuckeralkoholgemische enthält, gegebenenfalls zusammen mit Restmengen anderer Zuckeralkohole und Oligosaccharide.

Die erfindungsgemäßen Produkte können in Form von oralen Applikationsformen wie Pastillen, Tabletten, Komprimaten, Säften, Dragees, Hart- oder Weichkaramellen oder als Suspensionen, Sprays, Inhalationsgemischen oder ähnlichem vorliegen.

Die erfindungsgemäße Verwendung beziehungsweise die erfindungsgemäßen Produkte zeichnen sich also durch eine besonders zahnfreundliche Matrix in Form des erfindungsgemäß einzusetzenden Zuckeralkoholgemisches aus 1,1-GPM und 1,6-GPS, gegebenenfalls in Kombination mit 1,1-GPS, aus, wobei die gegebenenfalls vorhandenen Zink²⁺-Ionen nicht durch Komplexierung in ihrer Wirksamkeit gegen Erkältungskrankheiten bewirkende Mikroorganismen wie Rhinoviren oder als Immunstimulanz behindert werden. Die erfindungsgemäßen Produkte, insbesondere ausgeführt als Erkältungsmittel, beziehungsweise die erfindungsgemäße Verwendung zeichnen sich zudem durch ihre geringe Lösegeschwindigkeit im Vergleich zu bekannten Produkten aus, so daß der Wirkstoff über einen langen Zeitraum am gewünschten Wirkort abgegeben wird und einwirken kann. Schließlich sind die erfindungsgemäß einzusetzenden Zuckeralkoholgemische kalorienreduziert, auch für Diabetiker geeignet und erlauben die Herstellung von Produkten mit einer guten Lagerfähigkeit, so daß eine weniger aufwendige Verpackung ermöglicht wird. Aufgrund ihrer verbesserten Lagerfähigkeit zum Beispiel gegenüber zuckerhaltigen Produkten eignen sich die erfindungsgemäßen Produkte besonders auch für den Einsatz in feuchttropischen Gebieten, die häufig Gebiete mit Zinkmangel und hoher Infektionsgefahr sind.

Die Erfindung betrifft in einer weiteren Ausführungsform auch die vorgenannten Verwendungen und die vorgenannten zinkhaltigen Produkte, wobei das Zuckeralkoholgemisch ein entweder 1,6-GPS-angereichertes oder ein 1,1-GPM-angereichertes Gemisch ist, wie es in der DE 195 32 396 C2 beschrieben ist. Ein derartiges 1,6-GPS angereichertes Gemisch kann 1,6-GPS und 1,1-GPM in einem Verhältnis von 57 Gew.-% : 43 Gew.-% bis 99 Gew.-% : 1 Gew.-% (bezogen auf TS) enthalten. Ein derartiges 1,1-GPM angereichertes Gemisch kann beispielsweise 1,6-GPS und 1,1-GPM in einem Verhältnis von 1 Gew.-% : 99 Gew.-% bis 43 Gew.-% : 57 Gew.-% (bezogen auf TS) enthalten. Durch die erfindungsgemäß ermöglichte Variation der Mengenverhältnisse der eingesetzten Zuckeralkohole zueinander ist es möglich, erwünschte Lösegeschwindigkeiten der hergestellten Produkte einzustellen. Je nach gewünschter Anwendung und zu applizierendem Wirkstoff kann ein bestimmtes Verhältnis der Zuckeralkoholgemische zueinander eingestellt werden, so daß nahezu beliebige Lösegeschwindigkeiten und damit Freisetzungsgeschwindigkeiten der pharmakologisch wirksamen Bestandteile, insbesondere Zink, eingestellt werden können. Dies beruht darauf, daß sich die Lösekinetiken der Zukkeralkohole 1,6-GPS, 1,1-GPM und 1,1-GPS erheblich voneinander unterscheiden. So zeichnet sich 1,1-GPM durch eine deutlich geringere Lösegeschwindigkeit als 1,6-GPS oder anderen für diese Anwendung in Frage kommenden Substanzen ausiehe In Fällen, in denen eine rasche Freisetzung des Wirkstoffes, insbesondere des Zinks, erwünscht wird, werden erfindungsgemäß 1,6-GPS-angereicherte Gemische mit ihrer erhöhten Lösegeschwindigkeit eingesetzt, während in Fällen, in denen es um eine zeitlich verlängerte Freisetzung der Pharmazeutika ankommt, in erster Linie 1,1-GPM-angereicherte Gemische eingesetzt werden.

Demgemäß betrifft die Erfindung in einer bevorzugten Ausführungsform die Verwendung eines Zuckeralkoholgemisches aus mindestens 98 Gew.-% 1,6-GPS und 1,1-GPM, in der 75 bis 85 Gew.-% 1,6-GPS und 25 bis 15 Gew.-% 1,1-GPM enthalten sind und das als Isomalt GS bezeichnet wird. Zusätzlich können geringe Mengen an Mannit und Sorbit, zum Beispiel jeweils ≤ 0,5 Gew.-% in dem Zuckeralkoholgemisch vorhanden sein.

In einer weiteren bevorzugten Ausführungsform kann ein Zuckeralkoholgemisch vorgesehen sein, das bezogen auf die Gesamtmenge des Gemisches mehr als 90 Gew.-% 1,1-GPM und 1,6-GPS enthält, wobei Höchstmengen an Mannit von 3 Gew.-% und an Sorbit von 6 Gew.-% vorgesehen sein können. Ein solches Isomaltgemisch wird als Isomalt HC bezeichnet.

In einer weiteren bevorzugten Ausführungsform ist ein Zuckeralkoholgemisch vorgesehen, das mehr als 95 Gew.-% 1,6-GPS und 1,1-GPM aufweist, wobei 43 bis 57 Gew.-% davon 1,6-GPS sind. In dieser Ausführungsform ist vorgesehen, Höchstmengen an Mannit von 1 Gew.-% und Sorbit von 2 Gew.-% vorzusehen. Ein solches Isomalt wird als Isomalt HB bezeichnet.

Schließlich ist in einer weiteren bevorzugten Ausführungsform ein Zuckeralkoholgemisch vorgesehen, das mehr als 98 Gew.-% 1,6-GPS und 1,1-GPM aufweist, wovon 75 bis 85 Gew.-% 1,1-GPM und 25 bis 15 Gew.-% 1,6-GPS sind. In einer solchen Ausführungsform sind Höchstmengen an Mannit von 0,5 Gew.-% und an Sorbit von 0,5 Gew.-% vorgesehen. Dieses Zuckeralkoholgemisch wird als Isomalt GS bezeichnet.

Die vorgenannten Zuckeralkoholgemische können in der erfindungsgemäß vorgesehenen Verwendung und in den erfindungsgemäß vorgesehenen Arzneimitteln in vorteilhafter Weise eingesetzt werden, wobei besonders bevorzugt keine weiteren Süßungsmittel, Zucker oder Zuckeraustauschstoffe verwendet werden.

In einer besonders bevorzugten Ausführungsform der Erfindung ist vorgesehen, daß die erfindungsgemäßen Produkte beziehungsweise das erfindungsgemäß eingesetzte Zuckeralkoholgemisch aus 1,1-GPM und 1,6-GPS zusätzlich Intensiv-Süßstoffe wie Acesulfam-K, Aspartam, Cyclamat, Glycyrrhizin, Neotame, Neohesperidin DHC, Steviosid, Sucralose, Thaumatin, Saccharin oder ähnliche enthalten. In vorteilhafter Weise enthalten die erfindungsgemäßen Produkte beziehungsweise das erfindungsgemäß eingesetzte Zuckeralkoholgemisch aus 1,1-GPM und 1,6-GPS zudem Geschmacks- oder Aromastoffe wie Zitronen- oder Pfefferminz-Aroma. Die erfindungsgemäßen Produkte beziehungsweise das erfindungsgemäß eingesetzte Zukkeralkoholgemisch aus 1,1-GPM und 1,6-GPS können auch lebensmittelverträgliche Säuren wie Ascorbinsäure, Äpfelsäure oder Gluconsäure sowie als Gleitmittel Fettsäuren oder deren Salze wie Magnesiumstearat oder Natriumstearat enthalten. Schließlich kann vorgesehen sein, daß in den erfindungsgemäßen Produkten beziehungsweise im erfindungsgemäß eingesetzten Zuckeralkoholgemisch aus 1,1-GPM und 1,6-GPS Farbstoffe und/oder Sprengmittel, wie modifizierte Stärken, Polyvinylpyrrolidone (PVP) oder Carboxylmethylcellulose enthalten sind. Ein solches Gemisch wird als Isomalt GS bezeichnet.

Erfindungsgemäß können in bevorzugter Ausführungsform neben den erfindungsgemäß vorgesehenen Zuckeralkoholen 1,1-GPM und 1,6-GPS zusätzlich auch Enzyme, Coenzyme, Mineralstoffe, Vitamine, Antibiotika, Nikotin, Coffein, Eukalyptus, Codein, Phenacetin, Paracetamol, Acetylaminophenole, Acetylsalicylsäure, Menthol oder andere pharmakologisch aktive Wirkstoffe (vgl. Smit M.B.H. und Feldmann W. "Overthe-counter cold medications; siehe: A critical review of clinical trials between 1950 and 1991", in: JAMA 269, (1993) 2258-2263) in die Produkte oder Verwendungen eingeschlossen werden. Die pharmazeutisch aktiven Wirkstoffe, insbesondere Zink, sind in einer Menge vorzusehen, daß sie den erwünschten pharmakologischen Effekt bewirken.

Die Erfindung sieht in bevorzugter Ausführungsform auch vor, daß die 1,1-GPM und 1,6-GPS, insbesondere auch Zink, sowie gegebenenfalls 1,1-GPS und eventuell weitere vorgenannte Stoffe enthaltene Lebens-, Arznei- oder Genußmittel vollständig oder im wesentlichen zuckerfrei, das heißt insbesondere saccharose- und/oder fructose- und/oder glucosefrei sind. Unter im wesentlichen zuckerfrei wird verstanden, das maximal 10 Gew.-%, vorzugsweise maximal 5 Gew.-% und besonders bevorzugt maximal 1 Gew.-% Zucker (bezogen auf das Gesamtgewicht des Produktes) vorhanden sind.

Die erfindungsgemäßen Produkte zeichnen sich in der bevorzugten Ausführungsform durch ihre Zuckerfreiheit aus, sie sind zahnschonend, nicht kariogen und fördern darüber hinaus nicht die Bildung von Plaquematrices Zudem sind die erfindungsgemäßem Produkte im Unterschied zu leicht verdaulichen glucosehaltigen Produkten für Diabetiker geeignet, da Isomalt durch Dünndarmenzyme nur langsam und unvollständig gespalten wird und damit der Blutglucose-Spiegel kaum oder gar nicht steigt. Glykämischer Index und Insulin-Response sind entsprechend niedrig. Zudem wird in den Dickdarm gelangendes Isomalt zu kurzkettigen Fettsäuren fermentiert und wirkt damit Verstopfungen entgegen, woran insbesondere ältere Personen häufig leiden.

Weitere vorteilhafte Ausgestaltungen der Erfindung ergeben sich aus den Unteransprüchen.

Die Erfindung wird anhand von Beispielen und dazugehöriger Figuren näher erläutert. Es zeigen:
- Figur 1: den Einfluß von Isomalt und Zinksalz sowie von Vergleichssubstanzen auf das Wachstum von Staphylococcus aureus und
- Figur 2: das Absterbeverhalten von Staphylococcus aureus in 60 %iger Isomalt-Lösung (80 Teile 1,6-GPS : 20 Teile 1,1-GPM).

### Beispiele

### Beispiel 1: Antibakterieller Effekt von Isomalt

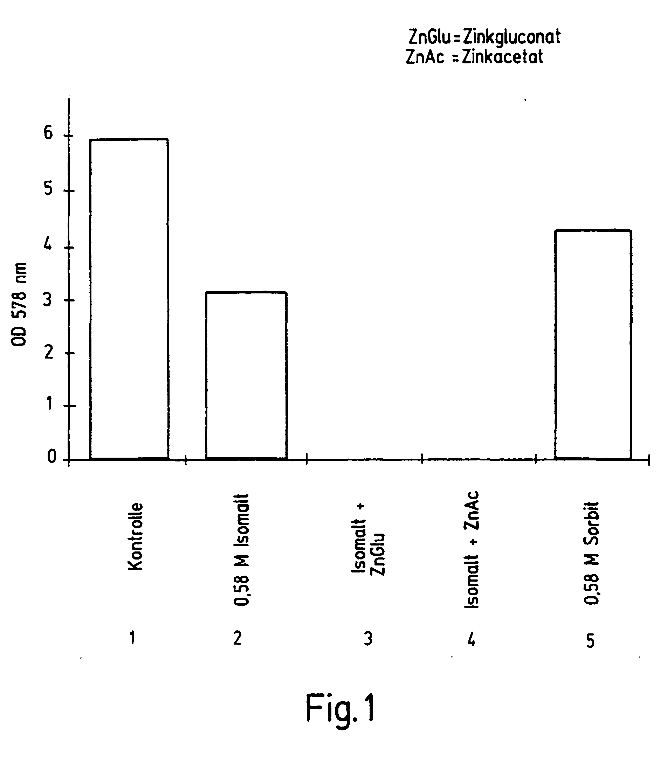

Staphylococcus aureus wurde bei 37° C in CASO-Medium kultiviert, dem neben der Kohlenstoffquelle Glucose (5 %ig) zusätzlich Isomalt(0,58 M) hinzugefügt worden war. Das Wachstum der Bakterienkulturen im Schüttelkolben wurde anhand der optischen Dichte (OD₅₇₈) nach 16 h bestimmt und mit einer Kontrolle (CASO-Medium ohne Isomalt) verglichen. Dabei wurde in allen Experimenten beobachtet, daß das Wachstum der in Isomalt angezogenen Kulturen um mehr als 50 % gehemmt wurde. Im Vergleich dazu wurde das Wachstum in äquimolaren Mengen Sorbit (0,58 M) lediglich auf etwa 75 % reduziert (s. Figur 1, Proben 1, 2 und 5).

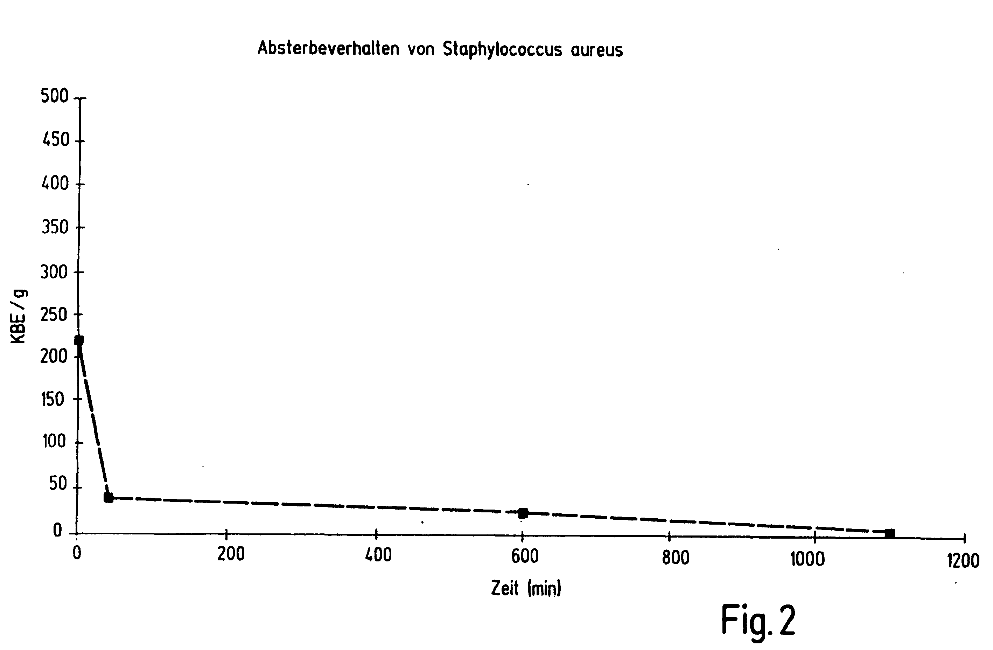

Weiterhin konnte ein antibakterieller Effekt einer 60 %igen 1,6-GPS angereicherten Lösung (80/20 Teile von 1,6-GPS/1,1-GPM) nachgewiesen werden (siehe Figur 2 für das Absterbeverhalten von Staphylococcus aureus).

Die hochkonzentrierte 60 %ige Isomalt-Lösung wurde mit etwa 1000 Keimen verschiedener Bakterien (Staphylococcus aureus und Streptoccocus pneumoniae) kontaminiert und es wurden zu unterschiedlichen Zeitpunkten Proben entnommen. Die in der Lösung überlebenden Keime wurden durch Filtration über ein Filter (0,45 µm) und Inkubation des Filters auf selektiven Agrarnährböden sichtbar gemacht. Es konnte gezeigt werden, daß bereits nach 30 min die Keimzahl erheblich reduziert war und nach einem Tag (1140 min) Bakterien fast nicht mehr nachweisbar waren.

### Beispiel 2: Antibakterieller Effekt von Zink/Isomalt

Isomalt-Hartkaramellen, in welche Zinkgluconat bzw. Zinkacetat (2,5 mg Zink pro g Isomalt) mit eingearbeitet worden waren, wurden 20 %ig (w/v) in CASO-Medium gelöst und mit einer frischen Staphylococcus aureus Kultur beimpft. Das Wachstum der Bakterien bei 37°C im Schüttelkolben wurde anhand der optischen Dichte (OD₅₇₈) nach 16 h bestimmt und mit einer Kontrolle (CASO-Medium ohne Hartkaramellen) verglichen. Weder die Kombination Isomalt-Zinkgluconat noch Isomalt-Zinkacetat ließ ein Wachstum der Bakterien zu. In allen Kulturen wurden optische Dichten von 0,0 gemessen (s. Figur 1, Proben 3 und 4).

### Beispiel 3: Bestimmung freier Zn-Ionen in Zukker-, Zuckeralkohol- und Hartkaramelllösen

10 ml Zinkgluconatlösung (2 g Zinkgluconat in 250 ml vollentsalztem Wasser) werden mit 10 ml Polyollösung (1.0 g Zucker bzw. Zuckeralkohol in 25 ml vollentsalztem Wasser gelöst) und 2 ml Acetatpuffer (pH = 6.0) gemischt und in einer Küvette auf 35 °C temperiert.

Anschließend wird 1 molare NaHCO₃-Lösung in kleineren Mengen zugetropft und mittels eines Trübungsphotometers die Trübung bestimmt.

In der Tabelle sind Isomalt, Saccharose, Glucose, Isomalt-Hartkaramellen, das als Zinkquelle eingesetzte Zinkgluconat sowie zwei Handelsprodukte gegenübergestellt, wobei die Gesamtmenge an Zn-Ionen jeweils 26 mg in 50 ml Lösung entspricht.

Freie Zinkionen und ihr prozentualer Anteil (der Gesamtmenge) zeigen, daß in Gegenwart von Isomalt (auch als Hartkaramelle) relativ viel freie Zinkionen im Vergleich zu anderen Polyolen verhanden sind.

**Tabelle:**

| | | |
|---|---|---|
| Nephelometrische Bestimmung freier Zn-Ionen in der Gegenwart von Polyolen (Handelsprodukt A: Hartkaramelle aus Saccharose-, Glucosesirup versetzt mit Zink-gluconat; Handelsprodukt B: Hartkaramelle aus Saccharose-, Glucosesirup versetzt mit Zinkgluconat und Zitronenaroma.) | | |

| Zucker/Zuckeralkohol/ Hartkaramelle | Zn-Ionen frei in* Lösung (in mg in 50 ml Lösung) | Prozentualer Anteil von freien (unkomplexierten) Ionen (in %) |
|---|---|---|
| Isomalt | 15,11 | 58 |
| Glucose | 13,00 | 50 |
| Saccharose | 12,40 | 48 |
| Isomalt/Hartkaramellen | 10,40 | 40 |
| Hartkaramellen Handelsprodukt A | 3,90 | 15 |
| Hartkaramellen Handelsprodukt B | 3,80 | 15 |
| Zn-Gluconat | 14,62 | 64 |

| | | |
|---|---|---|
| * Normiert auf gleiche Zn-Ionen-Konzentration | | |

### Beispiel 4: Antirhinovirale Aktivität von Isomalt-Zinkpräparaten

### Ausgangsmaterialien und Testverfahren:

Im Hinblick auf ihre mögliche antivirale Aktivität wurden vier verschiedene Zinkkonzentrationen (0,125 mM, 0,1 mM, 0,075 mM und 0,05 mM) in Form von Zinkgluconat und Zinkacetat zusammen mit 5 mg/ml Isomalt (äquimolares Gemisch aus 1,1-GPM und 1,6-GPS) getestet. Für jede der Kombinationen wurden drei identische Ansätze für 3 verschiedene Rhinovirus-Serotypen sowie jeweils eine Kontrolle unter Einschluß von EDTA (in äquimolarer Menge zu dem Zinksalz) durchgeführt. Weitere Kontrollen umfaßten Isomalt ohne Zinksalz und jede Zinksalzkonzentration ohne Isomalt.

Die genannten Präparate wurden in ihrer Wirkung auf Rhinoviren in HeLa-Zellen getestet (erhalten von der ATCC Rockville MD, Katalog-Nr. CRL-1958). Die HeLa Zellinie wurde in Dulbecco's modifiziertem Eagle-Medium (DMEM) kultiviert, supplementiert mit fötalem Rinderserum (5% Endkonzentration) und Gentamycinsulfat (50 µg/ml Endkonzentration). Die Zellkulturen wurden bei 37°C in feuchter 5%iger CO₂-Atmosphäre inkubiert. Bei Anwesenheit des Virus wurde die Inkubationstemperatur auf 33°C reduziert.

Die Rhinoviren-Stämme wurden auf HeLa-Zellen wachsen gelassen und die TCID₅₀ (Titer mit 50%iger Gewebekultur-Infektiösität) mittels reihenweiser Verdünnung ermittelt.

Die Zellen wurden unter Trypsin-Behandlung geerntet, gezählt und in jeder Vertiefung einer 96 Vertiefungen aufweisenden Mikrotiterplatte in einer Konzentration von 25.0000 Zellen pro Vertiefung gesät. Nach dem Impfen wurden die Platten über Nacht bei 37°C in feuchter 5%iger CO₂-Atmosphäre inkubiert und am nächsten Tag das Medium von den Zellen entfernt und mit 175 µl einer 1,143 x Konzentration der entsprechenden Testsubstanzkombination ersetzt. Die Platten wurden bei 37°C in feuchter 5%iger CO₂-Atmosphäre für eine Stunde inkubiert.

Nach dieser Inkubation wurden die Vertiefungen für die Bewertung der antiviralen Aktivität jeweils mit 25 µl einer DMEM-Lösung mit 100 TCID₅₀ des jeweiligen Virus versetzt. Pro Platten dienten 6 Vertiefungen als Virus-Kontrolle (ohne Zink-Isomalt-Präparat) und weitere 6 Vertiefungen als Zell-Kontrolle (ohne Zink-Isomalt-Präparat und ohne Virus). Die Platten wurden anschließend bei 33°C in feuchter 5%iger CO₂-Atmosphäre inkubiert und unter einem Mikroskop auf cytopathogene Effekte untersucht.

### Ergebnisse:

Die antivirale Aktivität wurde als die Verringerung des cytopathogenen Effekts von Rhinoviren in HeLa-Zellen bestimmt. Die Bestimmungen wurden mikroskopisch beziehungsweise unter Einsatz von colorimetrischen Tests durchgeführt. Die Bestimmung wurde zu dem Zeitpunkt vorgenommen, an dem die Viruskontrollen (ohne Zugabe von Zink-Isomalt-Präparaten) die volle Symptomatik eines viralen cytopathogenen Effektes zeigten. Für den Rhinovirustyp 7 wurde die Bewertung der antirhinoviralen Aktivität drei Tage nach der Infektion und für die Serumtypen 1 A und 2 vier Tage nach der Infektion durchgeführt. Die Zellkontrollen (HeLa-Zellen ohne Virus) zeigten keinerlei Symptome einer Virusinfektion, während die Viruskontrollen (HeLa-Zellen mit Virus ohne Zink-Isomalt-Präparat) die volle Symptomatik einer Virusinfektion zeigten.

Die virale Infektion von HeLa-Gewebekulturen führt zu histologischen Änderungen der Zellen. Diese können mikroskopisch beispielsweise durch Änderung der Zellform und der Desorption von Oberflächen beobachtet werden. Im Verlauf einer viralen Infektion können die Zellen nämlich zerstört und/oder vom Substrat abgelöst werden. Das Vorhandensein der Symptomatik einer viralen Infektion wurde vorliegend sowohl mikroskopisch als auch mittels Färbetechniken analysiert. Bei der Färbeanalyse macht man sich zunutze, daß sich infizierte Zellen vom Substrat ablösen und ausgewaschen werden. Vertiefungen mit Zellen, die gegen virale Infektion geschützt werden, weisen daher eine in Färbetests dunkler gefärbte Zellschicht auf, als Vertiefungen mit ungeschützten Zellen, da in letzteren weniger Zellen vorliegen, die zu einer Färbung der Zellschicht beitragen könnten.

Gegen den Rhinovirustyp 1a wurde mit 0,1 mM Zinkgluconat in Verbindung mit Isomalt antivirale Wirkung gegen Infektion durch Rhinovirustyp 1 A beobachtet. Ebenso wurde für 0,1 mM Zinkacetat in Verbindung mit Isomalt und 0,075 mM Zinkgluconat in Verbindung mit Isomalt eine antivirale Aktivität gegen den Rhinovirustyp 1 A erzielt. Diese Aktivitäten waren jedoch geringer als die mit 0,1 mM Zinkgluconat in Verbindung mit Isomalt erzielte.

Gegen den Rhinovirustyp 2 wurde mit 0,1 mM Zinkacetat zusammen mit Isomalt ebenso wie mit 0,1 mM Zinkgluconat in Verbindung mit Isomalt sowie, jeweils in schwächerem Ausmaß, mit 0,075 mM Zinkgluconat in Verbindung mit Isomalt sowie 0,1 mM Zinkgluconat ohne Isomalt ein antiviraler Effekt erzielt.

Gegen den Rhinovirustyp 7 wurde jeweils mit 0,05, 0,075 und 0,1 mM Zinkacetat in Verbindung mit Isomalt sowie 0,05 mM Zinkgluconat in Verbindung mit Isomalt ein antiviraler Effekt beobachtet, der im Ausmaß etwa dem Effekt von 0,075 mM Zinkgluconat in Verbindung mit Isomalt gegen Rhinovirus 2 entsprach.

Die antiviralen Aktivitäten gegen Rhinovirustyp 1a und 2 konnten durch Zugabe von EDTA in äquimolaren Mengen zu dem Zinksalz aufgehoben werden. Dies belegt die in der Beschreibungseinleitung diskutierte "freie Ionenhypothese". Zinkacetat und Zinkgluconat alleine ergaben nur einen vergleichsweise schwach ausgeprägten antiviralen Schutz durch 0,1 mM Zinkgluconat (ohne Isomalt). Der mit 0,1 mM Zinkgluconat erzielte Schutz war jedoch signifikant schwächer als der mit 0,1 mM Zinkacetat oder Zinkgluconat jeweils in Verbindung mit Isomalt erzielte Schutz.

Die Daten zeigen, daß pharmazeutische Präparate enthaltend Isomalt und Zinkgluconat oder Zinkacetat einen besseren Schutz gegen rhinovirale Infektionen bieten, als Zinkpräparate alleine.

## Patentansprüche

1. Verwendung eines 1,6-GPS-(6-O-a-D-Glucopyranosyl-D-sorbit) und 1,1-GPM-(1-O-a-D-Glucopyranosyl-D-mannit) haltigen Zuckeralkoholgemisches in Lebens-, Arznei- und Genußmitteln als therapeutischer Wirkstoff.

2. Verwendung nach Anspruch 1, wobei der therapeutische Wirkstoff ein antimikrobieller Wirkstoff ist.

3. Verwendung nach Anspruch 1, wobei der therapeutische Wirkstoff ein Immunstimulanz ist.

4. Verwendung nach einem der vorhergehenden Ansprüche, wobei 1,6-GPS und 1,1-GPM in dem Zuckeralkoholgemisch in nahezu äquimolaren Mengen vorhanden sind.

5. Verwendung nach einem der Ansprüche 1 bis 3, wobei das Zuckeralkoholgemisch ein 1,6-GPS oder 1,1-GPM angereichertes Gemisch ist.

6. Verwendung nach Anspruch 5, wobei in dem Zuckeralkoholgemisch 75 bis 85 Gew.-% 1,6-GPS und 25 bis 15 Gew.-% 1,1-GPM enthalten sind.

7. Verwendung nach Anspruch 5, wobei 75 bis 85 Gew.-% 1,1-GPM und 25 bis 15 Gew.-% 1,6-GPS in dem Zuckeralkoholgemisch vorhanden sind.

8. Verwendung nach einem der Ansprüche 1 bis 3 oder 5 bis 7, wobei das Zuckeralkoholgemisch zusätzlich 1,1-GPS (1-O-a-D-Glucopyranosyl-D-sorbit) enthält.

9. Verwendung nach einem der vorhergehenden Ansprüche, wobei das Zuckeralkoholgemisch zusätzlich Zink enthält.

10. Verwendung nach einem der vorhergehenden Ansprüche, wobei das Zink in Form von organischen oder anorganischen Zinksalzen vorliegt.

11. Verwendung nach einem der vorhergehenden Ansprüche, wobei das Zinksalz Zinkgluconat oder Zinkacetat ist.

12. Verwendung nach einem der vorhergehenden Ansprüche, wobei das Zuckeralkoholgemisch zusätzlich Farbstoffe, Aromen, Geschmacksstoffe, Genußsäuren oder geringe Anteile weiterer Zuckeralkohole enthält.

13. Verwendung nach einem der vorhergehenden Ansprüche, wobei das Lebens-, Genuß- oder Arzneimittel eine Hartkaramelle, eine Weichkaramelle, ein Komprimat, ein Dragee, eine Pastille oder ein Kaugummi ist.

14. Arzneimittel für den menschlichen oder tierischen Verzehr, enthaltend 1,1-GPM und 1,6-GPS sowie 0,5 - 10 mg Zink pro einem Gramm Zuckeralkohol.

15. Arzneimittel nach Anspruch 14, wobei das Arzneimittel zusätzlich 1,1-GPS enthält.

16. Arzneimittel nach einem der Ansprüche 14 oder 15, wobei das Arzneimittel 1,1-GPM und 1,6-GPS in nahezu äquimolaren Mengen enthält.

17. Arzneimittel nach einem der Ansprüche 14 oder 15, wobei das Arzneimittel 1,1-GPM und 1,6-GPS in Form von 1,1-GPM- oder 1,6-GPS-angereicherten Gemischen enthält.

18. Arzneimittel nach Anspruch 17, wobei das Arzneimittel 75 bis 85 Gew.-% 1,1-GPM und 25 bis 15 Gew.-% 1,6-GPS enthält.

19. Arzneimittel nach Anspruch 14 oder 15, wobei das Arzneimittel 15 bis 25 Gew.-% 1,1-GPM und 75 bis 85 Gew.-% 1,6-GPS enthält.

20. Arzneimittel nach einem der Ansprüche 14 bis 19, wobei Zink in Form von anorganischen oder organischen Zinksalzen vorliegt.

## Claims

1. Use of a sugar alcohol mixture containing 1,6-GPS-(6-O-α-D-glucopyranosyl-D-sorbitol) and 1,1-GPM-(1-O-α-D-glucopyranosyl-D-mannitol) as therapeutically active ingredient in foods, pharmaceutical compositions and semi-luxury foods.

2. Use according to claim 1, the therapeutically active ingredient being an antimicrobial active ingredient.

3. Use according to claim 1, the therapeutically active ingredient being an immunostimulant.

4. Use according to one of the preceding claims, 1,6-GPM and 1,1-GPM being present in the sugar alcohol mixture in almost equimolar quantities.

5. Use according to one of claims 1 to 3, the sugar alcohol mixture being a 1,6-GPS or 1,1-GPM enriched mixture.

6. Use according to claim 5, 75 to 85 % by wt. of 1,6-GPS and 25 to 15 % by wt. of 1,1-GPM being contained in the sugar alcohol mixture.

7. Use according to claim 5, 75 to 85 % by wt. of 1,1-GPM and 25 to 15 % by wt. of 1,6-GPS being present in the sugar alcohol mixture.

8. Use according to one of claims 1 to 3 or 5 to 7, the alcohol mixture additionally containing 1,1-GPS (1-O-α-D-glucopyranosyl-D-sorbitol).

9. Use according to one of the preceding claims, the sugar alcohol mixture additionally containing zinc.

10. Use according to one of the preceding claims, the zinc being present in the form of organic or inorganic zinc salts.

11. Use according to one of the preceding claims, the zinc salt being zinc gluconate or zinc acetate.

12. Use according to one of the preceding claims, the sugar alcohol mixture additionally containing colorants, aromas, flavourings, culinary acids or small proportions of further sugar alcohols.

13. Use according to one of the preceding claims, the food, semi-luxury food or pharmaceutical composition being a hard caramel, soft caramel, a compressed material, a coated pill, a lozenge or chewing gum.

14. Pharmaceutical composition for human or animal consumption containing 1,1-GPM and 1,6-GPS as well as 0.5 - 10 mg zinc per gram of sugar alcohol.

15. Pharmaceutical composition according to claim 14, the pharmaceutical composition additionally containing 1,1-GPS.

16. Pharmaceutical composition according to one of claims 14 or 15, the pharmaceutical composition containing 1,1-GPM and 1,6-GPS in almost equimolar quantities.

17. Pharmaceutical composition according to one of claims 14 or 15, the pharmaceutical composition containing 1,1-GPM and 1,6-GPS in the form of 1,1-GPM- or 1,6-GPS-enriched mixtures.

18. Pharmaceutical composition according to claim 17, the pharmaceutical composition containing 75 to 85 % by wt. of 1,1-GPM and 25 to 15 % by wt. of 1,6-GPS.

19. Pharmaceutical composition according to claim 14 or 15, the pharmaceutical composition containing 15 to 25 % by wt. of 1,1-GPM and 75 to 85 % by wt. of 1,6-GPS.

20. Pharmaceutical composition according to one of claims 14 to 19, zinc being present in the form of inorganic or organic zinc salts.

## Revendications

1. Utilisation d'un mélange d'alcools de sucres contenant du 1,6-GPS-(6-O-α-D-glucopyranosyl-D-sorbitol) et du 1,1-GPM-(1-O-α-D-glucopyranosyl-D-mannitol) comme agent thérapeutique dans les aliments, les médicaments et les produits d'agrément.

2. Utilisation selon la revendication 1, l'agent thérapeutique étant un principe actif anti-microbien.

3. Utilisation selon la revendication 1, l'agent thérapeutique étant un immuno-stimulant.

4. Utilisation selon l'une quelconque des revendications précédentes, le 1,6-GPS et le 1,1-GPM étant présents dans le mélange d'alcools de sucres en quantités à peu près équimolaires.

5. Utilisation selon l'une quelconque des revendications 1 à 3, le mélange d'alcools de sucres étant un mélange enrichi en 1,6-GPS ou 1,1-GPM.

6. Utilisation selon la revendication 5, le mélange d'alcools de sucres contenant 75 à 85 % en poids de 1,6-GPS et 25 à 15 % en poids de 1,1-GPM.

7. Utilisation selon la revendication 5, le mélange d'alcools de sucres contenant 75 à 85 % en poids de 1,1-GPM et 25 à 15 % en poids de 1,6-GPS.

8. Utilisation selon l'une quelconque des revendications 1 à 3, ou 5 à 7, le mélange d'alcools de sucres contenant en outre du 1,1-GPS (1-O-α-D-glucopyranosyl-D-sorbitol).

9. Utilisation selon l'une quelconque des revendications précédentes, le mélange d'alcools de sucres contenant en outre du zinc.

10. Utilisation selon l'une quelconque des revendications précédentes, le zinc étant présent sous forme de sels organiques ou inorganiques.

11. Utilisation selon l'une quelconque des revendications précédentes, le sel de zinc étant le gluconate de zinc ou l'acétate de zinc.

12. Utilisation selon l'une quelconque des revendications précédentes, le mélange d'alcools de sucres contenant en outre des colorants, des arômes, des agents de sapidité, des acides alimentaires ou de petites proportions d'autres alcools de sucres.

13. Utilisation selon l'une quelconque des revendications précédentes, l'aliment, le produit d'agrément ou le médicament étant un caramel dur, un caramel mou, un comprimé, une dragée, une pastille ou un chewing gum.

14. Médicament pour la consommation humaine ou animale contenant du 1,1-GPM et du 1,6-GPS ainsi que 0,5 à 10 mg de zinc pour 1 gramme d'alcools de sucres.

15. Médicament selon la revendication 14, ce médicament contenant en outre du 1,1-GPS.

16. Médicament selon l'une ou l'autre des revendications 14 ou 15, ce médicament contenant du 1,1-GPM et du 1,6-GPS en quantités à peu près équimolaires.

17. Médicament selon l'une ou l'autre des revendications 14 ou 15, ce médicament contenant du 1,1-GPM et du 1,6-GPS sous forme de mélanges enrichis en 1,1-GPM ou en 1,6-GPS.

18. Médicament selon la revendication 17, ce médicament contenant 75 à 85 % en poids de 1,1-GPM et 25 à 15 % en poids de 1,6-GPS.

19. Médicament selon la revendication 14 ou 15, ce médicament contenant 15 à 25 % en poids de 1,1-GPM et 75 à 85 % en poids de 1,6-GPS.

20. Médicament selon l'une quelconque des revendications 14 à 19, du zinc étant présent sous forme de sels inorganiques ou organiques.
